# EUROPEAN PATENT APPLICATION

(11) **EP 3 991 726 A1**
(43) Date of publication of application: **04.05.2022**
(21) Application number: 21206072.7
(22) Date of filing: 02.11.2021
(51) Int. Cl.: A61K 31/164, A61K 33/04, A61K 35/741, A61K 38/01, A61P 29/00

(54) **ANTI-INFLAMMATORY COMPOSITION COMPRISING PALMITOYLETHANOLAMIDE AND PHYCOCYANIN**

(30) Priority: 02.11.2020 IT 202000026062
(71) Applicant: Kura Srl, 10156 Torino (IT)
(72) Inventor: RAVETTI, Elena, 10156 TORINO (IT); RAVETTI, Guido, 10156 TORINO (IT); SILVAGNO, Maria Francesca, 10156 TORINO (IT)
(74) Representative: Bosia, Alessandra

(57) **Abstract**

The present invention concerns a composition comprising palmitoylethanolamide (PEA) and phycocyanin for use as an antiinflammatory agent, an antioxidant, and/or a protective agent for the mitochondrial metabolism.

## Description

### Cross-reference to related applications

This patent application claims priority from Italian patent application no. 102020000026062 filed on November 2, 2020.

### Technical field

The present invention concerns a composition comprising palmitoylethanolamide (PEA) and phycocyanin (PC) and uses thereof as an anti-inflammatory agent, antioxidant and/or protective agent for the mitochondrial metabolism. The composition is also used as a specific inhibitor of cyclooxygenase-2.

### State of the art

The causes of inflammation are numerous, including bacterial or viral infection which activates the immune system and results in the production of inflammatory cytokines and high levels of radical species which lead to oxidative damage. The metabolism of free radicals is a finely regulated process in the tissues, balanced between physiological production and endogenous protection systems (depending on glutathione). Malfunctioning of these processes can lead to the onset of inflammatory type tissue damage. Chronicization of the inflammatory phenomenon is one of the major causes of cell ageing, the development of some tumours, the onset of neurodegenerative and cardiovascular diseases. Diabetes has also been associated with chronic inflammation.

The intracellular signalling pathways that respond to inflammation are numerous, the most central one is mediated by activation of the NF-kB transcription factor. Reactive oxygen species (ROS) and activation of NF-kB are two inflammation mediators that stimulate each other. ROS come from environmental or pharmacological exposure, but they are produced in excess also by a metabolic disorder (for example malfunctioning of the mitochondrial metabolism); signals of the inflammatory cytokines, LPS (bacterial lipopolysaccharide) and other mediators that activate the TLR (e.g. TLR4) converge on the TLR (Toll Like Receptors) / NF-kB axis. NF-kB induces expression and activity of the cyclooxygenase 2 (COX-2) enzyme, which produces different derivatives of arachidonic acid such as prostaglandins (PG) . In particular PGE2 has a proinflammatory activity, it induces the production of inflammatory cytokines, and the two classes of pro-inflammatory molecules prostaglandins and cytokines activate NF-kB, in an amplification circle of the inflammatory response. The inhibition of COX-2 with nonsteroidal drugs reduces inflammation but has harmful consequences on simultaneous inhibition of the COX-1 enzyme, of physiological importance. More recently specific COX-2 inhibitors called coxibs (e.s. rofecoxib and celecoxib) have been introduced, however they have proved to be harmful as they are associated with an increased cardiovascular risk. The toxic effect appears to be due partly to the extreme selectivity and power in inhibiting COX-2, which accentuates the negative effects of the molecules produced by COX-1, and partly depends on the nonspecific action of these drugs on molecular targets other than COX-2 and interference with other signalling pathways that determine cardiotoxicity (Arora M. et al., 2020).

In addition to synthetic drugs, phycocyanin is proposed as a selective inhibitor of COX-2.

Phycocyanin is extracted from algae products such as klamath algae and spirulina algae.

To evaluate the therapeutic use of phycocyanin it is useful to consider its titer, namely the concentration of active substance.

In the starting algae products, this concentration can vary between 6% and 8%.

Furthermore, the phycocyanin contained in the algae has a very low level of bioavailability for the human organism. Extraction of phycocyanin is therefore necessary and this can be done with different methods: chemical procedures with the use of solvents, enzymatic procedures or physical procedures.

### Object of the invention

The object of the present invention is therefore to provide a new composition that acts as an anti-inflammatory agent, an antioxidant and/or protective agent of the mitochondrial metabolism (in particular antioxidant and/or protective agent of the mitochondrial metabolism), which is more effective and has fewer countereffects than the single and/or currently used products. A further object of the present invention is to provide a new composition that selectively inhibits cyclooxygenase-2 (COX-2).

According to the present invention, said object is achieved by means of the composition for use according to claim 1.

### Brief description of the figures

For a better understanding, the present invention will be described also with reference to the attached figures, which illustrate the following:

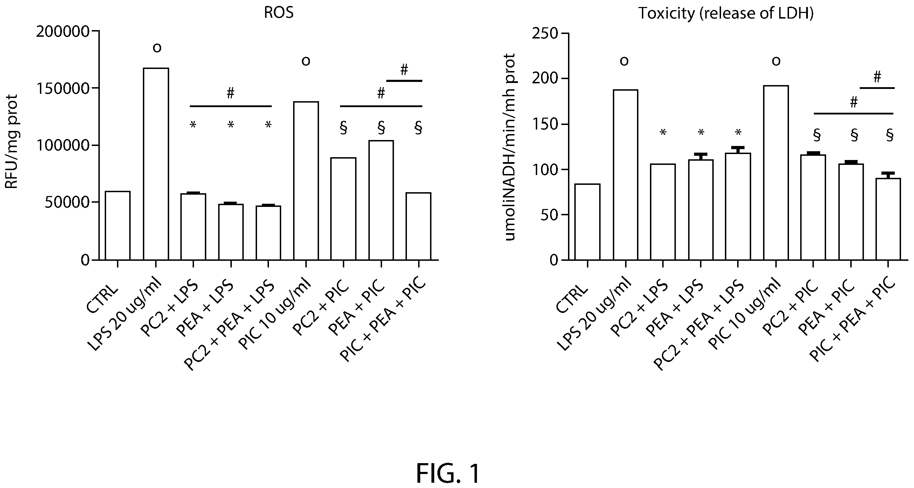

- Figure 1 shows a column graph illustrating the results of an experiment to evaluate protection against the toxic oxidising effect due to the production of reactive oxygen species (ROS) following stimulation with lipopolysaccharide (LPS) and Poly I:C (PIC) in the presence of PC, PEA and a combination thereof, measuring the fluorescence emission of the DCF-DA (dichlorofluorescein diacetate) intracellular probe;

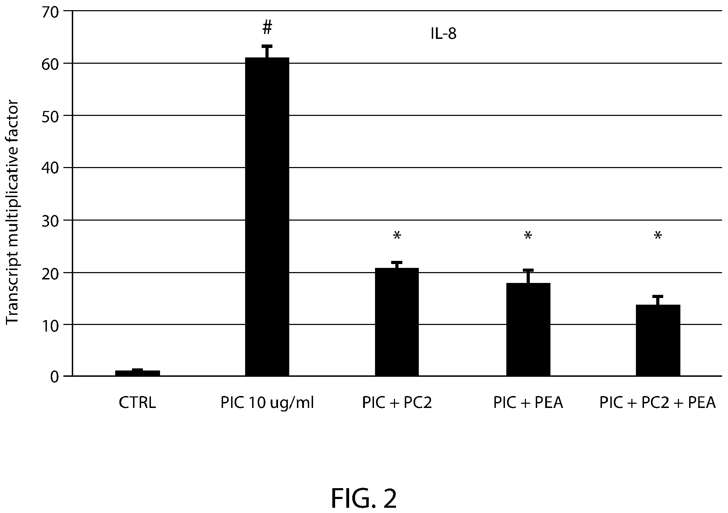

- Figure 2 shows a column graph illustrating the results of a real-time PCR experiment in which the interleukin 8 (IL-8) transcript is measured in cells treated with Poly I:C (PIC) and respectively phycocyanin, palmitoylethanolamide and both substances;

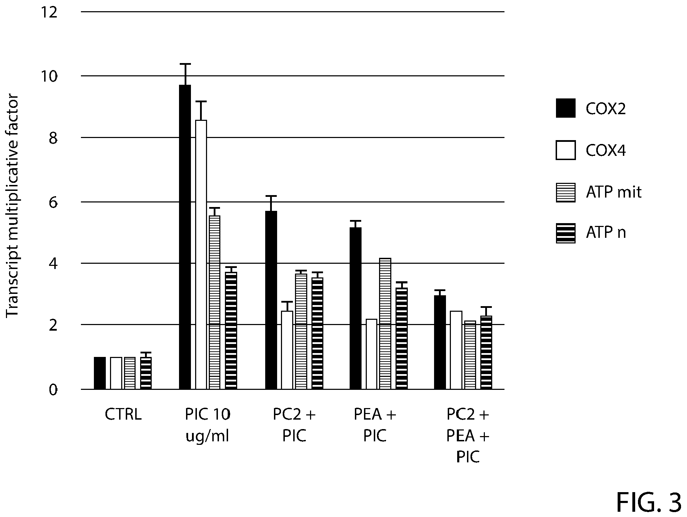

- Figure 3 shows a column graph illustrating the results of a real-time PCR experiment in which the following are measured: transcript for subunits 2 and 4 of the complex IV of the mitochondrial respiratory chain (COX2 and COX4), transcripts for two subunits of the ATP synthase mitochondrial enzyme (ATP mit and ATP n) in cells treated with Poly I:C (PIC) and respectively phycocyanin, palmitoylethanolamide and both substances;

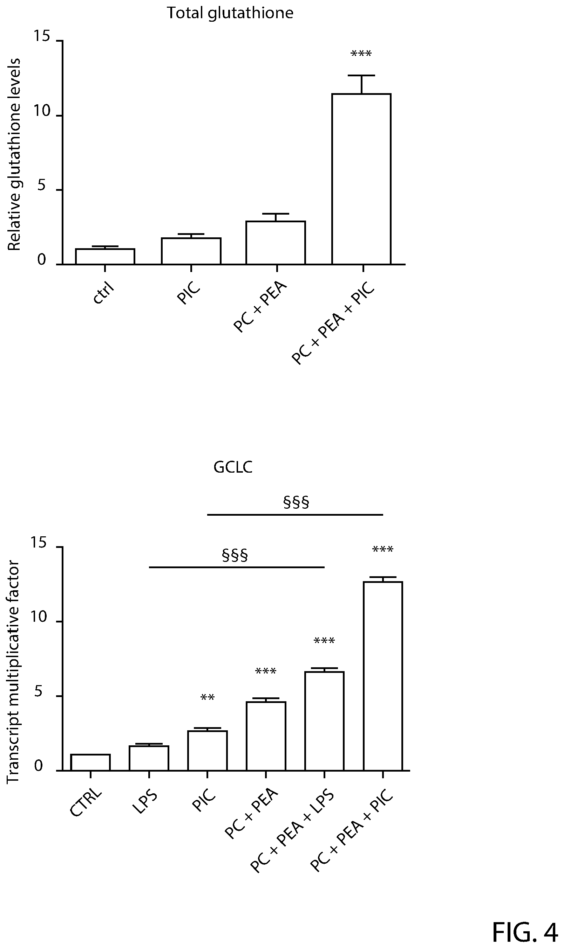

- Figure 4 shows a column graph illustrating the results of an intracellular glutathione and real-time PCR measurement experiment in which the transcript for the enzyme that synthesizes glutathione (GCLC) is measured.

### Detailed disclosure of the invention

The composition for use according to the present invention comprises phycocyanin and palmitoylethanolamide (PEA).

The phycocyanin used in the present invention is preferably extracted using a physical method such that it does not entail the possible presence of contaminating substances subsequent to the extraction.

The extract of phycocyanin can have concentrations of active substance of 10, 15, 20, 25% or more. In the present invention the use of blue phycocyanin with a titer equal to or higher than 25% is preferable.

Palmitoylethanolamide (PEA) is a substance known for its anti-inflammatory characteristics mediated by membrane receptors. In the present invention the association between phycocyanin and palmitoylethanolamide was surprisingly found to be particularly effective as an anti-inflammatory agent, as an anti-oxidant reducing the oxygen radicals and strengthening the antioxidant defences (for example, glutathione), and/or as a protective combination for protecting the mitochondrial metabolism. Furthermore, said association has been shown to strengthen inhibition of cyclooxygenase-2 (COX-2).

Inflammation is mediated partly by oxidative stress, but not necessarily or exclusively. The phycocyanin and palmitoylethanolamide combination acts in particular with an antioxidant effect.

With respect to the single substances, the association of phycocyanin and palmitoylethanolamide has a synergic and enhanced effect as shown below in the examples.

The composition preferably comprises PEA in a concentration from 25 to 45wt% and phycocyanin in a concentration from 35 to 55wt% of the total weight of the composition.

Even more preferably, the composition comprises PEA in a concentration from 30 to 40wt% and phycocyanin in a concentration from 40 to 50wt% of the total weight of the composition.

Even more preferably, the composition comprises PEA in a concentration of approximately 36.5wt% and phycocyanin in a concentration of approximately 45.5wt% of the total weight of the composition.

The composition preferably also comprises selenium or a probiotic, preferably *Bacillus Clausii.*

Selenium is necessary for the catalytic activity of glutathione peroxidase, the enzyme that uses glutathione to exert its antioxidant action in the tissues.

The composition preferably comprises at least one trace element, preferably selected from the group consisting of zinc, copper and selenium, and/or at least a further anti-inflammatory substance, preferably a natural alphalytic, even more preferably

*Serenoa repens.*

Preferably the composition is in the form of tablets or capsules. A preferred composition is the following.

550 mg capsule or tablet containing:
- 250 mg phycocyanin;
- 200 mg PEA;
- 55 µg selenium;
- excipients up to the weight of 550 mg.

The synergy between the two phycocyanin and PEA molecules allows use at lower concentrations than in other patented or marketed formulations. The composition is effective and is used as an anti-inflammatory agent, antioxidant and/or protective agent for protecting the mitochondrial metabolism, in particular in the treatment of inflammatory diseases of the respiratory system, the osteoarticular system, the nervous system, the urinary or reproductive system, and the eye.

Preferably, the diseases of the respiratory system are viral infections.

Preferably, the diseases of the osteoarticular system are osteoarticular pain.

Preferably, the diseases of the nervous system are headaches, dizziness or cognitive deficit.

Preferably, the diseases of the urinary system are prostatitis, urethritis or pelvic pain.

Preferably, the diseases of the reproductive system are male infertility.

The composition is also used as a selective inhibitor of cyclooxygenase-2 (COX-2).

### Examples

### Example 1 - Absence of cytotoxicity and absence of effect on proliferation

In a model of human pulmonary epithelium BEAS-2B (ATCC - CRL-9609), PC and PEA were tested in the range 10 nM - 1 µM. The absence of toxicity was verified by means of the MTT test (3,(4,5-dimethylthiazol-2)2,5 diphenyltetrazolium bromide) and no effect on cell growth was reported following proliferation after colouring with crystal violet.

### Example 2 - Inhibition of the enzyme COX-2

Measurement of the IC50 in an in vitro test (ELISA method - COX-2 (human) Inhibitor Screening Assay Kit, # 701080, Cayman Chemical) gave the following results.

| | IC50 (µM) |
|---|---|
| PC batch 1 | 0.3312 |
| PC batch 2 | 0.3645 |
| PEA | 0.5748 |
| PC batch 1 + PEA | 0.05624 |
| PC batch 2 + PEA | 0.03873 |

The IC50 values for PC are similar to what is reported in the literature. The PEA datum represents a complete novelty. The present results show:
- a direct inhibition activity of COX-2 by the PEA; and
- a synergic and enhanced action of PEA and phycocyanin on the inhibition of COX-2.

### Example 3 - Antioxidant effect on a model of human pulmonary epithelium: human pulmonary epithelium cell line BEAS-2B

As can be seen from Figure 1, PEA and phycocyanin (PC) protect from oxidative stress and toxicity induced by bacterial stimulus (LPS, lipopolysaccharide) or viral stimulus (PIC, Poly I:C, structurally similar to RNA with double viral helix). The PC+PEA combination brings the oxidative stress and toxicity values back to the control levels, and is more effective than the single substances.

Key to Figure: ° P < 0.05 vs CTRL; * P < 0.05 vs LPS; § P < 0.05 vs PIC; # P < 0.05 single vs combined treatment.

The cells were treated with PC and PEA 1 µM for 48h. The ROS (which are powerful oxidants) were measured as fluorescence emission of the intracellular probe DCF-DA (dichlorofluorescein diacetate). The toxicity was measured as release of an intracellular enzyme (lactate dehydrogenase LDH) in the extracellular medium; the release is proportional to the toxicity and is measured with a spectrophotometer.

### Example 4 - Anti-inflammatory effect on a model of human pulmonary epithelium: human pulmonary epithelium cell line BEAS-2B

As can be seen from Figure 2, PEA and phycocyanin (PC) reduce the production of inflammatory cytokines induced by bacterial or viral stimulus. For brevity, only the results relative to viral stimulus are illustrated. The results relative to bacterial stimulus (LPS) are equivalent.

The effect is enhanced by the PC+PEA combination.

The cells were treated with PC and PEA 1 µM for 24h. The interleukin 8 (IL-8) transcript was measured by means of real-time PCR.

Key to figure: #P < 0.05 vs CTRL; * P < 0.05 vs PIC.

### Example 5 - Effect of mitochondrial protection on a model of human pulmonary epithelium: human pulmonary epithelium cell line BEAS-2B

As can be seen from Figure 3, PC and PEA reduce activation of the mitochondrial respiratory burst induced by bacterial or viral stimulus. For brevity, only the results relative to viral stimulus are illustrated. The results relative to bacterial stimulus (LPS) are equivalent.

The effect is enhanced by the PC+PEA combination. This effect had never been described before either for the single molecules or for the combination thereof, and shows that the mixture of the two substances protects against mitochondrial oxidative damage.

The cells were treated with PC and PEA 1 µM for 24h. The transcripts for subunits 2 and 4 of complex IV of the mitochondrial respiratory chain (COX2 and COX4) and the transcripts for two subunits of the ATP synthase mitochondrial enzyme (ATP mit and ATP n) were measured by means of real-time PCR.

### Example 6 - Antioxidant effect mediated by glutathione on a model of human pulmonary epithelium: human pulmonary epithelium cell line BEAS-2B

As can be seen from figure 4, the treatment with phycocyanin and PEA in the presence of an inflammatory stimulus (PIC or LPS) increases the synthesis of glutathione. This is shown by the dosage of the total intracellular glutathione and by the measurement by means of real time PCR of the glutamyl-cysteine ligase (GCLC) enzyme that synthesizes glutathione. The data for dosage of intracellular glutathione and dosage of the enzyme responsible for synthesis of glutathione are consistent in showing that phycocyanin and PEA administered simultaneously increase the endogenous synthesis of glutathione, a very important molecule in natural defence of the tissues against oxidative stress and inflammation.

Key to Figure: ** P < 0.01 and *** P < 0.001 vs ctrl; §§§P < 0.001.

The cells were treated with PC at 25% and PEA 1 µM for 24h. The glutathione was measured with a colorimetric enzymatic method (Cayman Chemical, Michigan, USA) and the GCLC transcript was measured by means of real time PCR.

The antioxidant effect of the combination of phycocyanin and PEA is explained in its mechanism, namely reduction in production of the ROS (figure 1, example 3) and increase in the synthesis of glutathione, which is the natural antioxidant molecule of all the tissues (figure 4, example 6). Since the antioxidant and anti-inflammatory effect are closely connected, a new mechanism of the anti-inflammatory action of the two molecules is also shown.

The enhanced effect of the phycocyanin and PEA combination allows lower concentrations to be used than with the single molecules.

## Claims

1. A composition comprising palmitoylethanolamide (PEA) and phycocyanin for use as an anti-inflammatory agent, an antioxidant, and a protective agent for the mitochondrial metabolism.

2. A composition comprising palmitoylethanolamide (PEA) and phycocyanin for use as an antioxidant and a protective agent for the mitochondrial metabolism.

3. The composition for use according to claim 1 or 2, wherein phycocyanin is blue and has a titer equivalent to or higher than 25%.

4. The composition for use according to any of claims 1 to 3, wherein PEA is at a concentration from 25 to 45wt% and phycocyanin is at a concentration from 35 to 55wt% with respect to the total weight of the composition.

5. The composition for use according to any of claims 1 to 4, further comprising selenium.

6. The composition for use according to any of the preceding claims, further comprising a probiotic.

7. The composition for use according to claim 6, wherein the probiotic is *Bacillus Clausii.*

8. The composition for use according to any of the preceding claims, further comprising at least one trace element or at least one anti-inflammatory substance.

9. The composition for use according to any of the preceding claims in the form of a capsule or tablet.

10. The composition for use according to any of claims 1 to 9 in treating inflammatory diseases of the respiratory system, the osteoarticular system, the nervous system, the urinary or reproductive system, and the eye.

11. The composition for use according to any of the preceding claims, wherein the use is as a selective inhibitor of cyclooxygenase-2 (COX-2).
